# EUROPEAN PATENT SPECIFICATION

(11) **EP 4 007 449 B1**
(45) Date of publication and mention of the grant of the patent: **17.12.2025**
(21) Application number: 22151674.3
(22) Date of filing: 23.01.2018
(51) Int. Cl.: H05B 6/10, A24F 47/00

(54) **APPARATUS FOR HEATING SMOKABLE MATERIAL**
VORRICHTUNG ZUR ERWÄRMUNG VON RAUCHBAREM MATERIAL
APPAREIL DE CHAUFFAGE DE MATÉRIAU À FUMER

(30) Priority: 25.01.2017 US 201762450197 P
(43) Date of publication of application: 01.06.2022
(62) Divisional of application: 18704875.6
(73) Proprietor: Nicoventures Trading Limited, London WC2R 3LA (GB)
(72) Inventor: KAUFMAN, Duane A, Madison (US); PAPROCKI, Benjamin J, Madison (US)
(74) Representative: Dehns

(56) References cited:
- WO-A1-2014/140320
- WO-A1-2016/124550
- WO-A1-2017/001819
- US-A- 3 287 686
- US-A- 3 980 806
- US-A1- 2015 320 116
- US-A1- 2016 150 825

## Description

### TECHNICAL FIELD

The present invention relates to articles for use with apparatus for heating smokable material to volatilise at least one component of the smokable material, to apparatus for heating smokable material to volatilise at least one component of the smokable material, and to systems comprising such articles and such apparatus.

### BACKGROUND

Smoking articles such as cigarettes, cigars and the like burn tobacco during use to create tobacco smoke. Attempts have been made to provide alternatives to these articles by creating products that release compounds without combusting. Examples of such products are so-called "heat not burn" products or tobacco heating devices or products, which release compounds by heating, but not burning, material. The material may be, for example, tobacco or other non-tobacco products, which may or may not contain nicotine.

WO2017/001819 describes aerosol provision systems.

WO2016/124550 describes an improved extractor for an aerosol-generating device.

WO2014/140320 describes an aerosol-generating system with differential heating.

US2015/0320116 describes an apparatus according to the preamble of claim 4.

US2016/0150825 describes an aerosol-generating article with multi-material susceptor.

### SUMMARY

According to a first aspect, an apparatus for heating smokable material to volatilise at least one component of the smokable material is disclosed, in accordance with claim 1.

According to a second aspect, an apparatus for heating smokable material to volatilise at least one component of the smokable material is disclosed, in accordance with claim 4.

### BRIEF DESCRIPTION OF THE DRAWINGS

Embodiments of the invention will now be described, by way of example only, with reference to the accompanying drawings, in which:
Figure 1 shows a schematic perspective view of an example of a heating element for use with apparatus for heating smokable material to volatilise at least one component of the smokable material;
Figures 2 shows a plot of the paths of magnetic field lines through one half of the heating element of Figure 1 in use;
Figure 3 shows a graph of magnetic field strength as a function of distance along the length of the heating element of Figure 1 in use;
Figure 4 shows a schematic perspective view of an example of another heating element for use with apparatus for heating smokable material to volatilise at least one component of the smokable material;
Figure 5 shows a schematic cross-sectional view of the heating element of Figure 4;
Figure 6 shows a schematic cross-sectional view of an example of another heating element for use with apparatus for heating smokable material to volatilise at least one component of the smokable material;
Figure 7 shows a schematic cross-sectional view of an example of an article for use with apparatus for heating smokable material to volatilise at least one component of the smokable material, the article comprising the heating element of Figure 1;
Figure 8 shows a schematic cross-sectional view of an example of a system comprising the article of Figure 7, and apparatus for heating the smokable material of the article to volatilise at least one component of the smokable material;
Figure 9 shows a schematic cross-sectional view of an example of a system comprising an article comprising smokable material, and apparatus for heating the smokable material to volatilise at least one component of the smokable material, the apparatus including as an integral part the heating element of Figure 1;
Figure 10 shows a schematic cross-sectional view of an example of a system comprising an article comprising smokable material, and apparatus for heating the smokable material to volatilise at least one component of the smokable material, the apparatus including as an integral part the heating element of Figures 4 and 5;
Figure 11 shows a schematic cross-sectional view of an example of a system comprising an article comprising smokable material, and apparatus for heating the smokable material of the article to volatilise at least one component of the smokable material;
Figure 12 shows a schematic cross-sectional view of an example of another system comprising an article comprising smokable material, and apparatus for heating the smokable material of the article to volatilise at least one component of the smokable material; and
Figure 13 shows a schematic cross-sectional view of an example of a system comprising an article comprising smokable material and heating material, and apparatus for heating the smokable material of the article to volatilise at least one component of the smokable material.

### DETAILED DESCRIPTION

As used herein, the term "smokable material" includes materials that provide volatilised components upon heating, typically in the form of vapour or an aerosol. "Smokable material" may be a non-tobacco-containing material or a tobacco-containing material. "Smokable material" may, for example, include one or more of tobacco per se, tobacco derivatives, expanded tobacco, reconstituted tobacco, tobacco extract, homogenised tobacco or tobacco substitutes. The smokable material can be in the form of ground tobacco, cut rag tobacco, extruded tobacco, reconstituted tobacco, reconstituted smokable material, liquid, gel, gelled sheet, powder, or agglomerates, or the like. "Smokable material" also may include other, non-tobacco, products, which, depending on the product, may or may not contain nicotine. "Smokable material" may comprise one or more humectants, such as glycerol or propylene glycol.

As used herein, the term "heating material" or "heater material" refers to material that is heatable by penetration with a varying magnetic field.

Induction heating is a process in which an electrically-conductive object is heated by penetrating the object with a varying magnetic field. The process is described by Faraday's law of induction and Ohm's law. An induction heater may comprise an electromagnet and a device for passing a varying electrical current, such as an alternating current, through the electromagnet. When the electromagnet and the object to be heated are suitably relatively positioned so that the resultant varying magnetic field produced by the electromagnet penetrates the object, one or more eddy currents are generated inside the object. The object has a resistance to the flow of electrical currents. Therefore, when such eddy currents are generated in the object, their flow against the electrical resistance of the object causes the object to be heated. This process is called Joule, ohmic, or resistive heating. An object that is capable of being inductively heated is known as a susceptor.

It has been found that, when the susceptor is in the form of a closed electrical circuit, magnetic coupling between the susceptor and the electromagnet in use is enhanced, which results in greater or improved Joule heating.

Magnetic hysteresis heating is a process in which an object made of a magnetic material is heated by penetrating the object with a varying magnetic field. A magnetic material can be considered to comprise many atomic-scale magnets, or magnetic dipoles. When a magnetic field penetrates such material, the magnetic dipoles align with the magnetic field. Therefore, when a varying magnetic field, such as an alternating magnetic field, for example as produced by an electromagnet, penetrates the magnetic material, the orientation of the magnetic dipoles changes with the varying applied magnetic field. Such magnetic dipole reorientation causes heat to be generated in the magnetic material.

When an object is both electrically-conductive and magnetic, penetrating the object with a varying magnetic field can cause both Joule heating and magnetic hysteresis heating in the object. Moreover, the use of magnetic material can strengthen the magnetic field, which can intensify the Joule and magnetic hysteresis heating.

In each of the above processes, as heat is generated inside the object itself, rather than by an external heat source by heat conduction, a rapid temperature rise in the object and more uniform heat distribution can be achieved, particularly through selection of suitable object material and geometry, and suitable varying magnetic field magnitude and orientation relative to the object. Moreover, as induction heating and magnetic hysteresis heating do not require a physical connection to be provided between the source of the varying magnetic field and the object, design freedom and control over the heating profile may be greater, and cost may be lower.

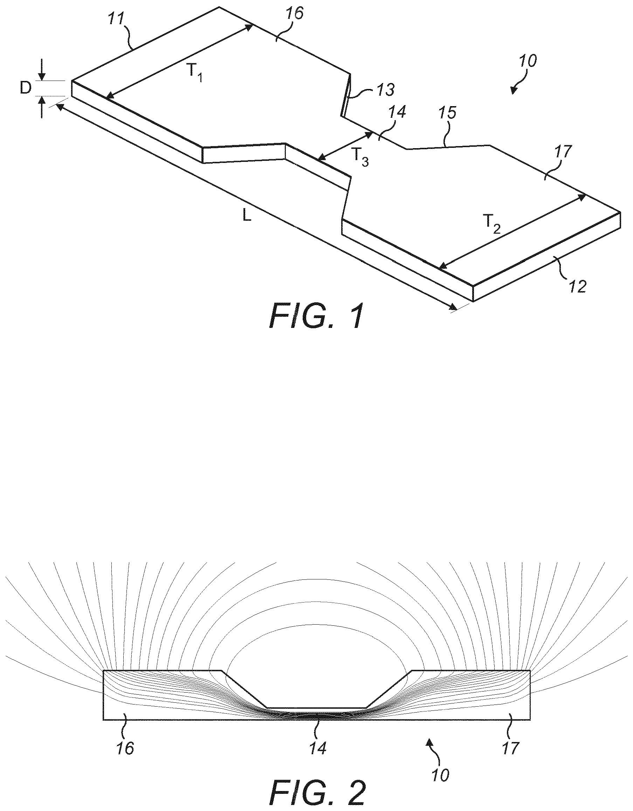

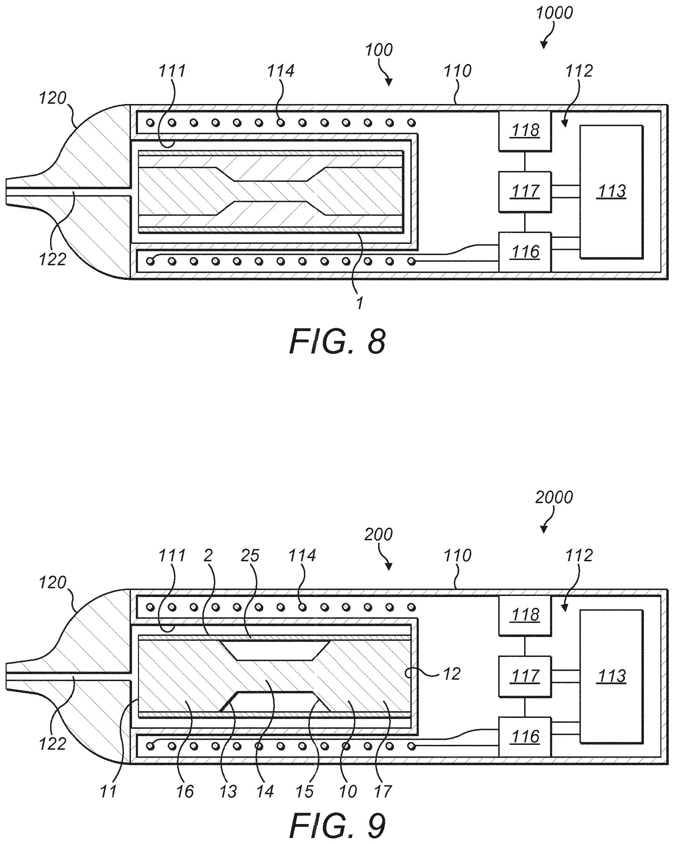

Referring to Figure 1, there is shown a schematic perspective view of an example of a heating element according to an embodiment of the invention. The heating element 10 is for use with apparatus for heating smokable material to volatilise at least one component of the smokable material, such as the apparatus 200 shown in Figure 9 and described below.

The heating element 10 is made of heating material that is heatable by penetration with a varying magnetic field. Further, in this embodiment the entire heating element 10 is made of a homogeneous, or substantially homogeneous, heating material, which in this embodiment is steel. The heating element 10 is a single-piece heating element 10, but in other embodiments the heating element 10 may instead comprise plural heating element parts that are joined or fixed to each other.

The heating element 10 has a first dimension and a second dimension perpendicular to the first dimension. The first dimension extends from a first portion of the heating element 10 to a second portion of the heating element 10. In this embodiment, the first dimension is a length L of the heating element 10, and the second dimension is a thickness T of the heating element 10. Moreover, in this embodiment, the first portion is a first longitudinal end 11 of the heating element 10, and the second portion is an opposite second longitudinal end 12 of the heating element 10. The heating element 10 also has a depth D that is perpendicular to each of the length L and the thickness T. The depth D is less than the length L and less than the thickness T.

The thickness T₃ at an intermediary section 14 of the heating element 10 that is spaced from the first and second longitudinal ends 11, 12 is less than the thickness T₁, T₂ at each of first and second sections 16, 17 that are located between the intermediary section 14 and the respective first and second longitudinal ends 11, 12. This reduced thickness is called a "geometric constriction" herein. In use, when the heating element 10 is penetrated with a varying magnetic field, the reduced thickness of the intermediary section 14 helps to increase the concentration of the field lines of the magnetic field in the intermediary section 14 of the heating element 10 as compared to the concentration of the field lines in the first and second sections 16, 17. This phenomenon is shown in Figure 2, which shows a plot of the paths of the magnetic field lines through one half of the heating element 10 of Figure 1 in use.

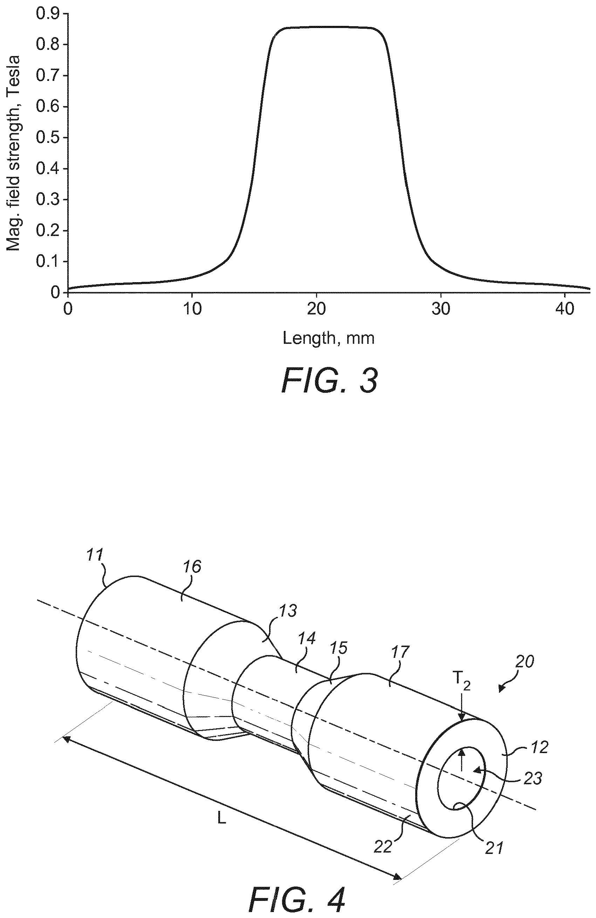

Figure 3 shows a graph of magnetic field strength as a function of distance along the length L of the heating element 10 of Figure 1 in use. From this graph, it can be seen that the magnetic field strength in the intermediary section 14 is considerably higher than the magnetic field strength in each of the first and second sections 16, 17. In this embodiment, the length L of the heating element 10 is about 42 millimetres, each of the thicknesses T₁, T₂ at the first and second sections 16, 17 is about 10 millimetres, and the thickness T₃ at the intermediary section 14 is about 2 millimetres. So, a ratio between the thickness T₃ at the intermediary section 14 and the thickness T₁, T₂ at each of the first and second sections 16, 17 is about 1:5. As can be seen from Figure 3, the magnetic field strength of a particular magnetic field with which the heating element 10 is penetrated is considerably higher (about eight times higher, in this embodiment) in the intermediary section 14 than in the first and second sections 16, 17.

In this embodiment, the thickness T₁, T₂, T₃ at each of the intermediary, first and second sections 14, 16, 17 is less than the length L. However, in some embodiments, the second dimension at one or each of the intermediary, first and second sections may instead be equal to or greater than the first dimension.

In some embodiments, the length L of the heating element may be between 30 and 50 millimetres such as between 30 and 40 millimetres. In some embodiments, the thickness T₁, T₂ at the first and/or second sections 16, 17 may be between 4 and 10 millimetres, such as between 5 and 8 millimetres. In some embodiments, the thickness T₃ at the intermediary section 14 may be between 0.4 and 2 millimetres, such as between 0.5 millimetres and 1 millimetre. In some embodiments, a ratio between the thickness T₃ at the intermediary section 14 and the thickness T₁, T₂ at one or each of the first and second sections 16, 17 may be between 1:5 and 1:25, such as between 1:7 and 1:15, for example 1:10.

The amount of energy deposited into the heating element 10 in use is a function of the magnetic field strength, among other factors such as excitation frequency. The amount of energy deposited into regions of the heating element 10 in which the magnetic field strength is relatively high will be greater than the amount of energy deposited into regions of the heating element 10 in which the magnetic field strength is relatively low. For example, in the present embodiment the magnetic field strength is increased by a factor of eight in the intermediary section 14 as compared to the first and second sections 16, 17. Therefore, the energy deposited per volume of the heating element 10 due to magnetic hysteresis heating will correspondingly be increased by a factor of eight in the intermediary section 14 as compared to the first and second sections 16, 17. The total energy per unit volume deposited may be higher, due to Joule heating in addition to magnetic hysteresis heating. Accordingly, using a heating element 10 with the geometry of embodiments of the present invention enables the intermediary section 14 of the heating element 10 to be heated to a greater temperature than the first and second sections 16, 17 of the heating element 10, by a given penetrating magnetic field.

Such varying temperature in different regions of a given heating element 10 may be used to provide a number of technical advantages in use.

For example, faster heating of the intermediary section 14 to a given temperature than the first and second sections 16, 17 may be used to provide progressive heating of smokable material in use. The relatively rapid heating of the intermediary section 14 may initiate volatilisation of at least one component of a first portion of a body of smokable material in thermal contact with the intermediary section 14 and formation of an aerosol therein. Over time, heating of the first and second sections 16, 17 may initiate volatilisation of at least one component of second and third portions of the body of smokable material in thermal contact with the first and second sections 16, 17 and formation of an aerosol therein.

In another example, when the heating element 10 is inductively heated using a magnetic field generator including an induction coil, the intermediary section 14 of the heating element 10 may reach the Curie point temperature of the heating material before the first and second sections 16, 17 reach that temperature. This will cause the magnetic permeability of the intermediary section 14 of the heating element 10 to drop towards near zero and essentially present an air-gap to the magnetic field. In turn, this will change the inductive load that the induction coil sees. A detector, such as an electronic circuit, may be configured to monitor the current in the induction coil to detect this phenomenon, and to control the inductive heating on the basis of the results of this monitoring.

The relative rates of heating the intermediary section 14 of the heating element 10 and the first and second sections 16, 17 of the heating element 10 may be controlled at least in part by selection of appropriate relative cross sectional areas of the intermediary section 14 and the first and second sections 16, 17.

In this embodiment, the thickness T₃ at the intermediary section 14 is less than a half of the thickness T₁, T₂ at each of the first and second sections 16, 17. Indeed, the thickness T₃ at the intermediary section 14 is less than a quarter of the thickness T₁, T₂ at each of the first and second sections 16, 17. In other embodiments, thickness T₃ at the intermediary section 14 may be more than a half or a quarter of the thickness T₁, T₂ at one or each of the first and second sections 16, 17.

In this embodiment, the thickness T₁ at the first section 16 is equal to the thickness T₂ at the second section 17. However, in other embodiments, this may not be true. For example, in some embodiments, the thickness T₁ at the first section 16 may be less than the thickness T₂ at the second section 17. Therefore, in some embodiments, the ratio between the thickness T₃ at the intermediary section 14 and the thickness T₁ at the first section 16 may be different from the ratio between the thickness T₃ at the intermediary section 14 and the thickness T₂ at the second section 17. This may further aid progressive heating of respective portions of any smokable material in thermal contact with the heating element 10 in use.

The heating element 10 of Figure 1 is planar, or substantially planar. In some variations to this embodiment, the heating element 10 may be other than planar.

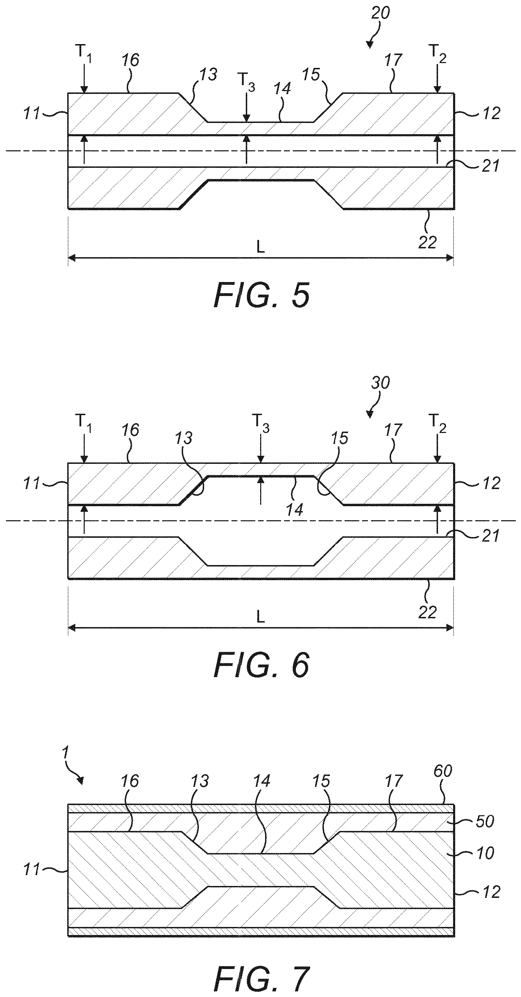

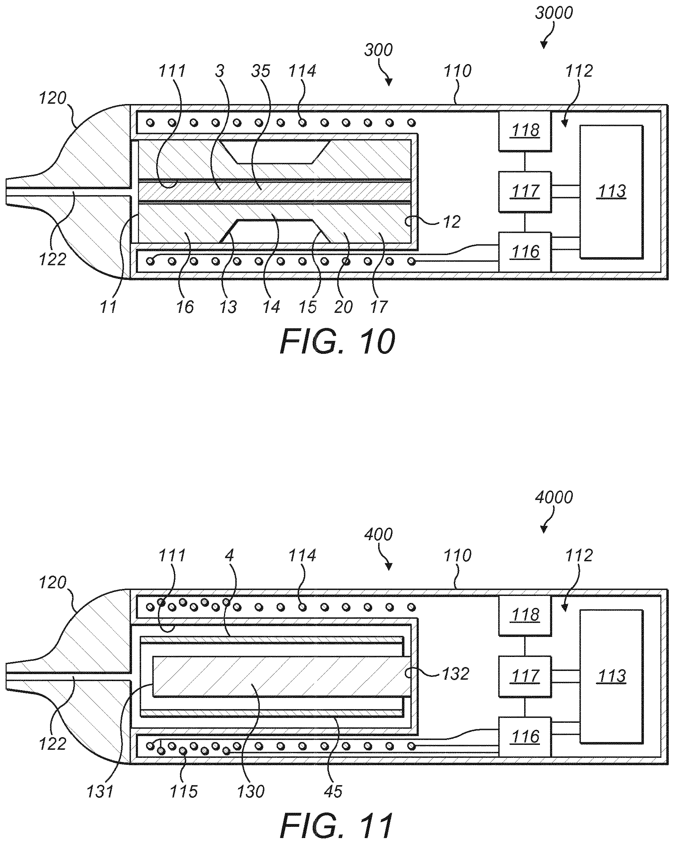

For example, referring to Figures 4 and 5, there are shown schematic perspective and cross-sectional views of an example of a heating element according to another embodiment of the invention. The heating element 20 is a tubular heating element 20 for use with apparatus for heating smokable material to volatilise at least one component of the smokable material, such as the apparatus 300 shown in Figure 10 and described below.

The heating element 20 again is made of heating material that is heatable by penetration with a varying magnetic field. Further, in this embodiment the entire heating element 20 is made of a homogeneous, or substantially homogeneous, heating material, which in this embodiment is steel. The heating element 20 is a single-piece heating element 20, but in other embodiments the heating element 20 may instead comprise plural heating element parts that are joined or fixed to each other.

The heating element 20 has a passageway 23 therein defined by an inner surface 21 of the heating element 20. In use, smokable material may be located in the passageway 23. The heating element 20 also has a first dimension and a second dimension perpendicular to the first dimension. The first dimension extends from a first portion of the heating element 20 to a second portion of the heating element 20. In this embodiment, the first dimension is an axial dimension of the heating element 20. More specifically, the first dimension is a length L of the heating element 20, the first portion is a first longitudinal end 11 of the heating element 20, and the second dimension is an opposite second longitudinal end 12 of the heating element 20. The passageway 23 has an opening at each of the first and second longitudinal ends 11, 12 of the heating element 20. In other embodiments, the passageway 23 may instead be closed at one or each of the first and second longitudinal ends 11, 12.

The second dimension is a thickness T of the heating element 20. The thickness T is measured radially from the inner surface 21 of the heating element 20 to an opposite outer surface 22 of the heating element 20, and in this embodiment in a direction normal to a longitudinal axis of the heating element 20. The thickness T₃ at an intermediary section 14 of the heating element 20 that is spaced from the first and second longitudinal ends 11, 12 is less than the thickness T₁, T₂ at each of first and second sections 16, 17 that are located between the intermediary section 14 and the respective first and second longitudinal ends 11, 12. In this embodiment, the thickness T₃ at the intermediary section 14 is less than a quarter of the thickness T₁, T₂ at each of the first and second sections 16, 17. In other embodiments, thickness T₃ at the intermediary section 14 may be, for example, less than a half and/or more than a quarter of the thickness T₁, T₂ at one or each of the first and second sections 16, 17. In this embodiment, the thickness T₁ at the first section 16 is equal to the thickness T₂ at the second section 17. However, in other embodiments, this may not be true.

In each of the heating elements 10, 20 of Figures 1, 2, 4 and 5, the intermediary section 14 is midway between the first and second longitudinal ends 11, 12 of the heating element 10. In other embodiments, the intermediary section 14 may be closer to the first longitudinal end 11 than to the second longitudinal end 12, or vice versa.

In each of the heating elements 10, 20 of Figures 1, 2, 4 and 5, the heating element 10, 20 comprises a first tapered region 13 in which the thickness T increases with distance from the intermediary section 14 towards the first section 16, and a second tapered region 15 in which the thickness T increases with distance from the intermediary section 14 towards the second section 17. The tapered regions 13, 15 help to reduce the concentration of stress between the intermediary section 14 and the first and second sections 16, 17 during heating of the heating element 10, 20. They also help provide a relatively smooth path for the magnetic field lines to follow in use. Nevertheless, in some embodiments, one or both of the tapered regions 13, 15 may be omitted.

The intermediary section 14 of the heating element 20 of Figures 4 and 5 has a shorter outer perimeter than each of the first and second sections 16, 17. In some variations to this embodiment, the intermediary section 14 may have a shorter outer perimeter than only one of the first and second sections 16, 17. In some embodiments, the intermediary section 14 may have a longer inner perimeter than one or each of the first and second sections 16, 17.

For example, referring to Figure 6, there is shown a schematic cross-sectional view of an example of a heating element according to another embodiment of the invention. The heating element 30 is a tubular heating element 30 for use with apparatus for heating smokable material to volatilise at least one component of the smokable material, such as a variation to the apparatus 300 shown in Figure 10 and described below. The heating element 30 of Figure 6 is identical to the heating element of Figures 4 and 5, except for the form of the intermediary section 14 and adjacent tapered regions 13, 15. The heating element 30 again has a first dimension and a second dimension perpendicular to the first dimension, wherein the first dimension extends from a first portion of the heating element 30 to a second portion of the heating element 30. Similarly to the heating element 20 of Figures 4 and 5, in this embodiment the first dimension is an axial dimension of the heating element 30. More specifically, the first dimension is a length L of the heating element 30, the first portion is a first longitudinal end 11 of the heating element 30, and the second dimension is an opposite second longitudinal end 12 of the heating element 30. However, in this embodiment, the thickness T₃ of the intermediary section 14 is less than the thickness T₁, T₂ at each of first and second sections 16, 17 by way of the intermediary section 14 having an outer perimeter than matches those of the first and second sections 16, 17, and a longer inner perimeter than each of the first and second sections 16, 17.

In some other variations (not shown) to the embodiments described above, the heating element is tubular, the first dimension of the heating element is a diameter of the heating element, the first and second portions of the heating element are diametrically opposed sides of the heating element, and the second dimension perpendicular to the first dimension is an axial dimension of the heating element. In some such embodiments, the heating element is elongate and the axial dimension is a length of the heating element. In some embodiments, the tubular heating element is made of a homogeneous, or substantially homogeneous, heating material. The heating element may be a single-piece heating element.

In these variations, the tubular heating element has an intermediary section that is spaced, circumferentially, from the diametrically opposed sides of the heating element. Further, the heating element has first and second sections that are located, circumferentially, between the intermediary section and the respective diametrically opposed sides. The heating element is provided with an axial geometric constriction, so that the axial dimension of the intermediary section of the heating element is less than the axial dimension at each of first and second sections. The axial dimension at the intermediary section may, for example, be less than a half or less than a quarter of the axial dimension at one or each of the first and second sections. Similarly to the heating elements of Figures 1 and 4 to 6, the heating element may have a first tapered region in which the axial dimension increases with distance from the intermediary section towards the first section, and may further have a second tapered region in which the axial dimension increases with distance from the intermediary section towards the second section.

In some such embodiments, the axial geometric constriction is spaced from both axial ends of the heating element. For example, the axial geometric constriction may be midway between the axial ends. In other embodiments, the geometric constriction is spaced only from a first axial end of the heating element, so that it is located at the opposite second axial end of the heating element.

A heating element 10, 20, 30 embodying the present invention may be provided as part of an article for use with apparatus for heating smokable material to volatilise at least one component of the smokable material, the article comprising the heating element and smokable material in thermal contact with the heating element.

For example, referring to Figure 7 there is shown a schematic cross-sectional view of an example of an article for use with apparatus for heating smokable material to volatilise at least one component of the smokable material. The article 1 is for use with apparatus for heating smokable material to volatilise at least one component of the smokable material, such as the apparatus 100 shown in Figure 8 and described below.

The article 1 comprises the heating element 10 of Figure 1, smokable material 50 in thermal contact with the heating element 10, and a cover 60 around the smokable material 50. For conciseness, the heating element 10 will not be described again in detail.

In this embodiment, the smokable material 50 is in surface contact with the heating element 10. More specifically, respective portions of the smokable material 50 are in surface contact with the intermediary section 14 and the first and second sections 16, 17 of the heating element 10. This is achieved by adhering the smokable material 50 to the heating element 10. However, in other embodiments, the fixing may be by other than adhesion. In some embodiments the smokable material 50 may not be fixed to the heating element 10 as such. The surface contact may help heat to be conducted directly from the heating element 10 to the smokable material 50. In other embodiments, the heating element 10 may be kept out of surface contact with the smokable material 50. For example, in some embodiments, the article 1 may comprise a thermally-conductive barrier that is free from heating material and that spaces the heating element 10 from the smokable material 50 of the article 1 in use. In some embodiments, the thermally-conductive barrier may be a coating on the heating element 10. The provision of such a barrier may be advantageous to help to dissipate heat to alleviate hot spots in the heating element 10.

In this embodiment, the heating material of the heating element 10 has a Curie point temperature that is less than the combustion temperature of the smokable material 50. The combustion temperature may be the autoignition temperature or kindling point of the smokable material 50. That is, the lowest temperature at which the smokable material 50 will spontaneously ignite in normal atmosphere without an external source of ignition, such as a flame or spark.

Accordingly, when the temperature of the heating element 10 in use reaches the Curie point temperature, the ability to further heat the heating element 10 by penetration with a varying magnetic field is reduced or removed. For example, as noted above, when the heating material is electrically-conductive, Joule heating may still be effected by penetrating the heating material with a varying magnetic field. Alternatively, when the heating material is non-electrically-conductive, depending on the chemical composition of the heating material, such further heating by penetration with a varying magnetic field may be impossible.

Thus, in use, this inherent mechanism of the heating material of the heating element 10 may be used to limit or prevent further heating of the heating element 10, so as to help avoid the temperature of the adjacent smokable material 50 from reaching a magnitude at which the smokable material 50 burns or combusts. Thus, in some embodiments, the chemical composition of the heating element 10 may help enable the smokable material 50 to be heated sufficiently to volatilise at least one component of the smokable material 50 without burning the smokable material 50. In some embodiments, this may also help to prevent overheating of the apparatus with which the article 1 is being used, and/or help to prevent part(s), such as the cover 60 or an adhesive, of the article 1 being damaged by excessive heat during use of the article 1.

In some embodiments, if the combustion temperature of the smokable material 50 is greater than X degrees Celsius, then the chemical composition of the heating material may be provided so that the Curie point temperature is no more than X degrees Celsius. For example, if the combustion temperature of the smokable material 50 is greater than 300 degrees Celsius, then the chemical composition of the heating material may be provided so that the Curie point temperature is no more than 300 degrees Celsius. The Curie point temperature may be, for example, less than 300 degrees Celsius, less than 280 degrees Celsius, less than 260 degrees Celsius, less than 240 degrees Celsius, or less than 220 degrees Celsius.

In some embodiments, the ability of the heating material to be heated by penetration with a varying magnetic field by magnetic hysteresis heating may return when the temperature of the heating material has dropped below the Curie point temperature.

In this embodiment, the cover 60 encircles the smokable material 50. The cover 60 helps to protect the smokable material 50 from damage during transport and use of the article 1. During use, the cover 60 may also help to direct the flow of air into and through the smokable material 50, and may help to direct the flow of vapour or aerosol through and out of the smokable material 50.

In this embodiment, the cover 60 comprises a wrapper that is wrapped around the smokable material 50 so that free ends of the wrapper overlap each other. The wrapper thus forms all of, or a majority of, a circumferential outer surface of the article 1. The wrapper may be formed from paper, reconstituted tobacco, or the like. The cover 60 also comprises an adhesive (not shown) that adheres the overlapped free ends of the wrapper to each other. The adhesive may comprise one or more of, for example, polyvinyl acetate (PVA), gum Arabic, natural or synthetic resins, starches, and varnish. The adhesive helps prevent the overlapped free ends of the wrapper from separating. In other embodiments, the adhesive may be omitted.

The cover 60 defines an outer surface of the article 1 and may contact the apparatus in use. In this embodiment, the article 1 is elongate and cylindrical with a substantially circular cross-section. However, in other embodiments, the article 1 may have a cross-section other than circular and/or not be elongate and/or not be cylindrical. In this embodiment, the heating element 10 extends from a first longitudinal end of the smokable material 50 to an opposite second longitudinal end of the smokable material 50. This need not be the case in other embodiments. In this embodiment, the heating element 10 and smokable material 50 extend from a first longitudinal end of the article 1 to an opposite second longitudinal end of the article 1. This need not be the case in other embodiments.

In some embodiments, the article 1 may comprise a thermal wick to aid measurement of a temperature of the smokable material 50 at a location radially-inwards of the cover 60, such as at the interface between the smokable material 50 and the cover 60.

In the embodiment of Figure 7, the article 1 comprises the heating element 10 of Figure 1. In some other embodiments, the article may alternatively or additionally comprise the heating element 20 of Figures 4 and 5 and/or the heating element 30 of Figure 6 and/or one of the herein-described heating elements, such as the herein-described variations to the heating elements 10, 20, 30 of Figures 1, 4, 5 and 6.

Referring to Figure 8, there is shown a schematic cross-sectional view of an example of a system according to an embodiment of the invention. The system 1000 comprises the article 1 of Figure 7, and apparatus 100 for heating the smokable material 50 of the article 1 to volatilise at least one component of the smokable material 50. In the interest of conciseness, the article 1 will not be described again in detail. Any of the herein-described possible variations to the article 1 of Figure 7 may be made to the article 1 of the system 1000 of Figure 8 to form separate respective embodiments of a system. Similarly, the article 1 of Figure 7 may be replaced in the system 1000 by one of the other articles described above to form separate respective embodiments of a system. The apparatus 100 comprises a heating zone 111 for receiving the article 1, and a device 112 for heating the heating element 10 of the article 1 when the article 1 is located in the heating zone 111.

The apparatus 100 of this embodiment comprises a body 110 and a mouthpiece 120. The mouthpiece 120 defines a channel 122 therethrough. The mouthpiece 120 is locatable relative to the body 110 so as to cover an opening into the heating zone 111. When the mouthpiece 120 is so located relative to the body 110, the channel 122 of the mouthpiece 120 is in fluid communication with the heating zone 111. In use, the channel 122 acts as a passageway for permitting volatilised material to pass from the article 1 inserted in the heating zone 111 to an exterior of the apparatus 100. In this embodiment, the mouthpiece 120 of the apparatus 100 is releasably engageable with the body 110 so as to connect the mouthpiece 120 to the body 110. In other embodiments, the mouthpiece 120 and the body 110 may be permanently connected, such as through a hinge or flexible member. In some embodiments, such as embodiments in which the article itself comprises a mouthpiece or the body 110 comprises the channel 122, the mouthpiece 120 of the apparatus 100 may be omitted.

The apparatus 100 defines an air inlet that fluidly connects the heating zone 111 with the exterior of the apparatus 100. The air inlet may be defined by the body 110 of the apparatus 100 and/or by the mouthpiece 120 of the apparatus 100. A user may be able to inhale the volatilised component(s) of the smokable material 50 by drawing the volatilised component(s) through the channel 122 of the mouthpiece 120. As the volatilised component(s) are removed from the article 1, air may be drawn into the heating zone 111 via the air inlet of the apparatus 100.

In this embodiment, the body 110 comprises the heating zone 111. In this embodiment, the heating zone 111 comprises a recess for receiving the article 1. The article 1 may be insertable into the heating zone 111 by a user in any suitable manner, such as through a slot in a wall of the apparatus 100, or by first moving a part of the apparatus, such as the mouthpiece 120, to access to the heating zone 111. In other embodiments, the heating zone 111 may be other than a recess, such as a shelf, a surface, or a projection, and may require mechanical mating with the article in order to co-operate with, or receive, the article. In this embodiment, the heating zone 111 is sized and shaped to accommodate the whole article 1. In other embodiments, the heating zone 111 may be dimensioned to receive only a portion of the article 1 in use.

The device 112 comprises a magnetic field generator 112 for generating the varying (such as alternating) magnetic field for penetrating the heating element 10 of the article 1 in use.

In this embodiment, the magnetic field generator 112 comprises an electrical power source 113, a coil 114, a device 116 for passing a varying electrical current, such as an alternating current, through the coil 114, a controller 117, and a user interface 118 for user-operation of the controller 117.

In this embodiment, the electrical power source 113 is a rechargeable battery. In other embodiments, the electrical power source 113 may be other than a rechargeable battery, such as a non-rechargeable battery, a capacitor or a connection to a mains electricity supply.

In some embodiments, the electrical power source 113 is a DC power source, and the apparatus 100 comprises a DC/AC inverter connected to the DC power source. The DC/AC inverter may comprise a Class-E power amplifier.

The coil 114 may take any suitable form. In this embodiment, the coil 114 is a helical coil of electrically-conductive material, such as copper. In this embodiment, the cross-section of the coil 114 is circular, but in other embodiments it may be other than circular, such as square. In some embodiments, the magnetic field generator 112 may comprise a magnetically permeable core around which the coil 114 is wound. Such a magnetically permeable core concentrates the magnetic flux produced by the coil 114 in use and makes a more powerful magnetic field. The magnetically permeable core may be made of iron, for example. In this embodiment, the coil 114 encircles the heating zone 111. In this embodiment, the coil 114 extends along a longitudinal axis that is substantially aligned with a longitudinal axis of the heating zone 111. In this embodiment, the coil 114 is a helical coil of circular-cross-section electrically-conductive material, but in other embodiments the cross-sectional shape may be other than circular, such as flat or oblong. The coil 114 could be plated, e.g. silver-plated.

In some embodiments, the impedance of the coil 114 may be matched with the impedance of the heating element 10. This may be achieved, for example, by appropriate selection of the number of turns of the coil 114, the spacing between the heating element 10 and the coil 114, the heating material used for the heating element 10, capacitance of the drive circuit, and/or the electrical resistance of the susceptor current path.

In this embodiment, the device 116 for passing a varying current through the coil 114 is electrically connected between the electrical power source 113 and the coil 114. In this embodiment, the controller 117 also is electrically connected to the electrical power source 113, and is communicatively connected to the device 116 to control the device 116. More specifically, in this embodiment, the controller 117 is for controlling the device 116, so as to control the supply of electrical power from the electrical power source 113 to the coil 114. In this embodiment, the controller 117 comprises an integrated circuit (IC), such as an IC on a printed circuit board (PCB). In other embodiments, the controller 117 may take a different form. In some embodiments, the apparatus may have a single electrical or electronic component comprising the device 116 and the controller 117. The controller 117 is operated in this embodiment by user-operation of the user interface 118. The user interface 118 is located at the exterior of the body 110. The user interface 118 may comprise a push-button, a toggle switch, a dial, a touchscreen, or the like.

In this embodiment, operation of the user interface 118 by a user causes the controller 117 to cause the device 116 to cause an alternating electrical current to pass through the coil 114, so as to cause the coil 114 to generate an alternating magnetic field. The coil 114 and the heating zone 111 are relatively positioned so that, when the article 1 is located in the heating zone 111, the alternating magnetic field produced by the coil 114 penetrates the heating material of the heating element 10 of the article 1. When the heating material of the heating element 10 is an electrically-conductive material, as in this embodiment, this may cause the generation of one or more eddy currents in the heating material. The flow of eddy currents in the heating material against the electrical resistance of the heating material causes the heating material to be heated by Joule heating. Further, when the heating material is made of a magnetic material, as in this embodiment, the orientation of magnetic dipoles in the heating material changes with the changing applied magnetic field, which causes heat to be generated in the heating material.

In use, when the heating element 10 is inductively heated using the magnetic field generator 112 including the induction coil 114, the intermediary section 14 of the heating element 10 reaches the Curie point temperature of the heating material before the first and second sections 16, 17 reach that temperature. As discussed above, this causes the magnetic permeability of the intermediary section 14 of the heating element 10 to drop towards near zero and essentially present an air-gap to the magnetic field. In turn, this will change the inductive load that the induction coil 114 sees. The device 116 of the magnetic field generator 112 comprises a detector for detecting an electrical current in the coil 114, and the controller 117 is configured to control operation of the magnetic field generator 112 on the basis of a change in the electrical current detected by the detector. That is, on the basis of one or more signals received from the detector, the controller 117 may adjust a characteristic of the varying or alternating electrical current passed through the coil 114. The characteristic may be, for example, amplitude or frequency or duty cycle.

The controller 117 may, for example, be arranged to cause generation of the varying magnetic field to cease when the intermediary section 14 of the heating element 10 has reached the Curie point temperature more than a predetermined number of times, such as one, two, three, five or ten times, or when it is determined that the heating element 10 is about to reach the Curie point temperature. In some embodiments, the controller 117 may be arranged to monitor the power required to drive the magnetic field generator 112. The controller 117 may be arranged to detect the power used as the heating element 10 heats up to its Curie point temperature. The controller 117 may be arranged to determine the power required to maintain that known temperature, which is an indication of a condition (such as water content or volatile content) of the smokable material. In some embodiments, the controller 117 may be arranged to cause the temperature of the heating element 10 to be maintained at or just below the Curie point temperature.

So, as discussed above, the Curie point temperature of the heating material can be used to inherently prevent or hinder induction heating of the heating element 10 above that temperature, to ensure that the temperature of the smokable material 50 remains within a predetermined temperature range. Within the predetermined temperature range, in use the smokable material 50 is heated sufficiently to volatilise at least one component of the smokable material 50 without combusting the smokable material 50. Accordingly, the controller 117, and the apparatus 100 as a whole, is arranged to heat the smokable material 50 to volatilise the at least one component of the smokable material 50 without combusting the smokable material 50. In some embodiments, the temperature range is about 50°C to about 350°C, such as between about 50°C and about 250°C, between about 50°C and about 150°C, between about 50°C and about 120°C, between about 50°C and about 100°C, between about 50°C and about 80°C, or between about 60°C and about 70°C. In some embodiments, the temperature range is between about 170°C and about 220°C. In other embodiments, the temperature range may be other than this range. In some embodiments, the upper limit of the temperature range could be greater than 300°C. Additionally, the intermediary section 14 of the heating element 10 reaching the Curie point temperature in use may cause the controller 117 to effect a change in the way the magnetic field generator 112 operates.

In some embodiments, the apparatus 100 may comprise an additional temperature sensor (not shown) for sensing a temperature of the heating element 10 and sending a signal comprising an identifier of the magnitude of the temperature to the controller 117. The controller 117 may control the magnetic field generator 112 at least in part on the basis of the received signal. The controller 117 could comprise a proportional-integral-derivative (PID) controller that adjusts the power provided to the device 16 on the basis of a detected error in heating element 10 temperature, determined by comparison of the sensed magnitude of the temperature and the Curie point temperature of the heating material of the heating element 10.

In some embodiments, the apparatus could comprise a capacitor or capacitor discharge unit controlled, e.g. by the controller 117, to help provide a rapid increase in current through the coil 114 in use, to decrease the time a user would need to wait to effect for initiation of volatilisation of at least one component of the smokable material 50. Alternative or additionally, the apparatus could comprise a temperature sensor (not shown) for detecting the latent temperature of the smokable material before use of the system, in a sensitive enough manner to detect the latent-heat plateau, and to then momentarily or rapidly increase the temperature of the heating element on the basis of this detected latent temperature, to decrease the time a user would need to wait to effect for initiation of volatilisation of at least one component of the smokable material 50.

In some embodiments, the controller 117 may run software that is configured to accommodate manufacturing variations in electronic components, e.g. to accommodate vulnerabilities in how a specific circuit performs in use.

In some embodiments, the controller 117 may be configured to control the output power to the coil 114, in case of supply voltage change, by use of a buck regulator or a choke regulator.

In the system 1000 of Figure 8, the heating element 10 is part of the article 1, and the apparatus 100 is free from heating elements 10 arranged for penetration by the varying magnetic field generated by the magnetic field generator 112. However, in other embodiments, the apparatus itself comprises such a heating element.

For example, referring to Figure 9 there is shown a schematic cross-sectional view of an example of another system according to an embodiment of the invention. The system 2000 comprises an apparatus 200 and an article 2. The system 2000 of Figure 9 is identical to the system 1000 of Figure 8 except for the form of the article and except that the apparatus 200, rather than the article 2, comprises the heating element 10 of Figure 1. Like features with the system 1000 of Figure 8 retain the same reference numerals in Figure 9. Any of the herein-described possible variations to the system 1000 of Figure 8 may be made to the system 2000 of Figure 9 to form separate respective embodiments of a system.

The article 2 is tubular and comprises smokable material 25. The heating element 10 projects into the heating zone 111. More specifically, in this embodiment, the first longitudinal end 11 of the heating element 10 is a free end that is arranged relative to the heating zone 111 so as to enter a central cavity of the tubular article 2 as the article 2 is inserted into the heating zone 111. On the other hand, the second longitudinal end 12 of the heating element 10 is directly attached or fixed to the rest of the apparatus 200.

Referring to Figure 10 there is shown a schematic cross-sectional view of an example of another system according to an embodiment of the invention. The system 3000 comprises an apparatus 300 and an article 3. The system 3000 of Figure 10 is identical to the system 2000 of Figure 9 except for the form of the article, and the forms of the heating zone and heating element of the apparatus. Like features with the system 2000 of Figure 9 retain the same reference numerals in Figure 10. Any of the herein-described possible variations to the systems described above may be made to the system 3000 of Figure 10 to form separate respective embodiments of a system.

In the embodiment of Figure 10, the article 3 is rod-shaped and comprises smokable material 35. The heating element of the apparatus 300 is the heating element 20 of Figures 4 and 5. The heating element 20 extends around the heating zone 111 so that the passageway 23 of the heating element 20 effectively is the heating zone 111. In variations to this embodiment, the heating element 20 may be altered so that it extends only partially around the heating zone 111.

In each of the systems 2000, 3000 of Figures 9 and 10, the device 112 of the apparatus 200, 300 is for heating the heating element 10, 20 of the apparatus 200, 300. In turn, the heating element 10, 20 of the apparatus 200, 300 is for heating the heating zone 111. More specifically, each of the devices 112 of the apparatuses 200, 300 of Figures 9 and 10 is the same as the device 112 of the apparatus 100 of Figure 8 except that, rather than being arranged to inductively heat an inductive-heatable portion of an article located in the heating zone 111, each of the devices 112 of the apparatuses 200, 300 of Figures 9 and 10 is used to inductively heat the heating element 10, 20 of the apparatus 200, 300. The heat generated in the heating element 10, 20 of the apparatus 200, 300 passes to the article 2, 3 in the heating zone 111 in use by way of heat conduction.

Accordingly, the article 2, 3 with which the apparatus 200, 300 is usable need not comprise material that is readily inductively heatable to heat the smokable material 25, 35 to a temperature sufficient to volatilise at least one component of the smokable material 25, 35. This may enable the article 2, 3 to be made of cheaper or more readily-available material.

In the above-described systems, the shape of the heating element causes the strength of a magnetic field that penetrates the heating element to differ at respective different locations in the heating element. However, in other embodiments, it is the configuration of the magnetic field generator of the apparatus that causes this effect.

For example, referring to Figure 11 there is shown a schematic cross-sectional view of an example of another system according to an embodiment of the invention. The system 4000 comprises an apparatus 400 and an article 4. The system 4000 of Figure 11 is identical to the system 2000 of Figure 9 except for the form of the heating element of the apparatus, and except that the magnetic field generator 112 comprises a second coil 115 that surrounds part of the first coil 114. Like features with the system 2000 of Figure 9 retain the same reference numerals in Figure 11. Any of the herein-described possible variations to the systems described above may be made to the system 4000 of Figure 11 to form separate respective embodiments of a system.

The heating element 130 of the apparatus 400 of this embodiment is free from any geometric constriction like that of the heating elements 10, 20, 30 discussed above. Rather, in this embodiment, the heating element 130 has first and second longitudinal ends 131, 132, a length that extends from the first longitudinal end 131 to the opposite second longitudinal end 132, and a constant cross-sectional shape and size along that length. The cross-sectional shape may be, for example, polygonal, square or circular.

The article 4 is tubular and comprises smokable material 45. The heating element 130 projects into the heating zone 111. More specifically, the first longitudinal end 131 of the heating element 130 is a free end that is arranged relative to the heating zone 111 so as to enter a central cavity of the tubular article 4 as the article 4 is inserted into the heating zone 111. On the other hand, the second longitudinal end 132 of the heating element 130 is directly attached or fixed to the rest of the apparatus 400. A nano-coating or cover layer may be provided on the heating element 130 to help prevent fouling of the heating element 130. In a variation to this embodiment, the heating element 130 may have a male thread for cooperating with a female thread formed at the interior of the tubular article 4, so that the article 4 may be screwed onto the heating element 130 in use.

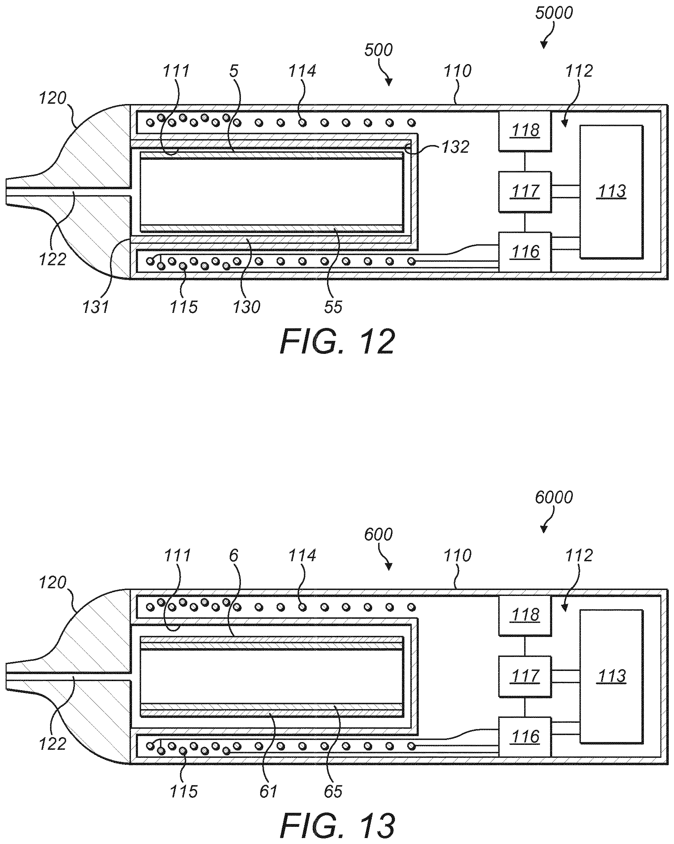

Referring to Figure 12 there is shown a schematic cross-sectional view of an example of another system according to an embodiment of the invention. The system 5000 comprises an apparatus 500 and an article 5. The system 5000 of Figure 12 is identical to the system 4000 of Figure 11 except for the forms of the heating zone and heating element of the apparatus. Like features with the system 4000 of Figure 11 retain the same reference numerals in Figure 12. Any of the herein-described possible variations to the systems described above may be made to the system 5000 of Figure 12 to form separate respective embodiments of a system.

The heating element 130 of the apparatus 500 extends around the heating zone 111. In variations to this embodiment, the heating element 130 may be altered so that it extends only partially around the heating zone 111.

Referring to Figure 13 there is shown a schematic cross-sectional view of an example of another system according to an embodiment of the invention. The system 6000 comprises an apparatus 600 and an article 6. The system 6000 of Figure 13 is identical to the system 5000 of Figure 12 except for the form of the article, and in that the apparatus is free from heating elements arranged for penetration by the varying magnetic field generated by the magnetic field generator 112. Like features with the system 5000 of Figure 12 retain the same reference numerals in Figure 13. Any of the herein-described possible variations to the systems described above may be made to the system 6000 of Figure 13 to form separate respective embodiments of a system.

The article 6 is tubular and comprises a tubular heating element 61 of heating material, and a tube of smokable material 65 that is fixed to an inner surface of the heating element 61 so as to be in thermal contact with the heating material. In other embodiments, the smokable material 65 may be fixed to an outer surface of the heating element 61, or the article may take a different form, such as rod-shaped. In each of these embodiments, however, the article 6 comprises both a heating element comprising heating material, and smokable material in thermal (preferably surface) contact with the heating material.

In variations to this embodiment, the article 6 may take one of many other possible forms. For example, the article 6 could comprise a heating element in the form of a body of "wire wool" or steel wool, which may surround a core comprising smokable material. Alternatively, the heating element of the article 6 may take the form of an open cell foam, or be deposited, on the smokable material or on a further carrier, using vapour deposition. Alternatively, the article 6 may comprise one or more spherical heating elements of heating material, which may be hollow, and the spherical heating elements may be wrapped in smokable material. Alternatively, the article 6 may comprise a plurality of heating element filaments of heating material, which may be located in different densities in different locations within the article 6. The heating element of the article 6 may include one or more surface irregularities or bumps, to decrease uneven heating, or may be in the form of axially-short cylinders. The article 6 could instead comprise ferrite dispersed in the smokable material, so that the ferrite acts as a plurality of heating elements. In still further embodiments, the article takes the form of a laminated tablet, with a first layer of the laminate comprising smokable material and a second layer of the laminate comprising a heating element of heating material. A hole may be formed through the article in a direction normal to the layers, for the release and direction of aerosol generated in the article in use. In some embodiments, the heating element could be formed using one or more manufacturing techniques that enables the creation of a greater surface area, such as between the smokable material and heating material. Such techniques comprise, but are not limited to, additive manufacturing (3D printing) and casting, such as lost wax casting.

In each of the apparatuses 400, 500, 600 of the systems of Figures 11 to 13, the magnetic field generator 112 is for generating plural varying magnetic fields that penetrate the heating element 61, 130 in use in such a way that a strength of a sum of the magnetic fields differs at respective different locations in the heating element 61, 130.

More specifically, in each of the embodiments of Figures 11 to 13, the magnetic field generator 112 comprises a first coil 114 for generating a first of the varying magnetic fields, and a second coil 115 for generating a second of the varying magnetic fields, wherein the second varying magnetic field overlaps the first varying magnetic field in use. In these embodiments, the second varying magnetic field only partially overlaps the first varying magnetic field in use. The device 116 is electrically connected between the electrical power source 113 and the second coil 115, to enable the device 116 to pass a varying current through the second coil 115.

Each of the first and second coils 114, 115 is a helical coil but, in another embodiment, one or each of the coils 114, 115 may take a different form. Further, each of the first and second helical coils extends along an axis, and the axes of the first and second coils 114, 115 in each embodiment are coincident. Accordingly, the two helical coils 114, 115 are coaxial. However, in other embodiments, the axes of the two helical coils 114, 115 may instead be parallel or inclined to each other.

In each of these embodiments, the second coil 115 surrounds the first coil 114. That is, the second coil 115 has a larger radius that the first coil 114. Further, in each of these embodiments, the second coil 115 surrounds only a section of the first coil 114, rather than the whole of the first coil 114. In each of these embodiments, the section is less than half of the length of the first coil 114.

In some embodiments, the controller 117 controls the device 116 to cause the device 116 to pass respective varying currents through the coils 114, 115 simultaneously, and so that the strength of a sum of the magnetic fields generated by the first and second coils 114, 115 is greater at a first part of the heating element 61, 130 than at a second part of the heating element 61, 130. In use, this would initiate volatilisation of at least one component of the smokable material 45, 55, 65 adjacent the first part of the heating element 61, 130 and formation of an aerosol therein, before initiation of volatilisation of at least one component of the smokable material 45, 55, 65 adjacent the second part of the heating element 61, 130 and formation of an aerosol therein. Accordingly, there is provided progressive heating of the smokable material 45, 55, 65 of the article 4, 5, 6 over time.

In these embodiments, the section of the first coil 114 surrounded by the second coil 115 is a section closest to the channel 122 of the apparatus. This helps to enable an aerosol to be formed and released relatively rapidly from the article 4, 5, 6 at a location relatively close to the channel 122, for inhalation by a user, yet provides time-dependent release of aerosol, so that aerosol continues to be formed and released even after the smokable material 45, 55, 65 adjacent the first part of the heating element 61, 130 has ceased generating aerosol. Such cessation of aerosol generation may occur as a result of the smokable material 45, 55, 55 becoming exhausted of volatilisable components.

In some embodiments, the apparatus may comprise the two coils 114, 115 in the relative arrangement described above with reference to Figures 11 to 13, and further the heating element may comprise a geometric constriction, such as one of the geometric constrictions described above with reference to Figures 1 to 6. The heating element may be comprised in the apparatus or in an article for use with the apparatus.

In some embodiments, the apparatus may comprise a puff detector (not shown), for detecting when a user is drawing fluid through the apparatus via the channel 122 and sending a signal representing the detected puff to the controller 117. In some such embodiments, the controller 117 may control the device 116 to cause the device 116 to pass respective varying currents through each of the coils 114, 115 at respective times in dependence on the signal received from the puff detector. For example, the controller 117 may control the device 116 to cause the device 116 to change which of the two coils 114, 115 the device 116 is passing a current through, in dependence on a count of a number of puffs detected. Such a regime could again be used to provide progressive heating. In some embodiments, cooling of the heating element or smokable material in use may cause the temperature of the heating element to drop below its Curie point temperature. The controller 117 may be arranged to determine that a user is drawing fluid through the apparatus on the basis of this detected drop.

A section of the first coil 114 with a relatively small cross-sectional area may create a higher strength magnetic field than a section of the first coil 114 with a relatively large cross-sectional area. Accordingly, in some embodiments, the first coil 114 may have a varying cross-sectional area along its axial length, so as to create a higher strength magnetic field in a first part of the heating element 61, 130 than in a second part of the heating element 61, 130. The cross-sectional area of the coil 114 may taper from large to small along the axial length of the coil 114. In some such embodiments, the second coil 115 may be omitted, so that the magnetic field generator 112 is for generating only one varying magnetic field that penetrates the heating element 61, 130 in use.

In some embodiments, the article 1, 2, 3, 4, 5, 6 may have a lip sensor (not shown) for detecting contact with the lips of a user. The lip sensor may comprise, for example, a piezoelectric device, a pressure sensor, or a sensor configured such that electrical conductivity of the lip sensor differs when the user's lips are in contact with the lip sensor as compared to when the lips are out of contact with the lip sensor. The lip sensor may be communicatively connected to the controller 117 when the article 1, 2, 3, 4, 5, 6 is in the heating zone 111, so that the controller 117 may effect an action on the basis on a signal received from the lip sensor.

In each of the above described embodiments, there is only a single geometric constriction in the heating element 10, 20, 30. In other embodiments, the heating element may comprise plural geometric constrictions, which may differ from each other. For example, the thickness of the heating element at one of the constrictions may be different to the thickness of the heating element at another one of the constrictions. In some such embodiments, in use a first of the constrictions (e.g. the constriction with the least thickness) may reach the Curie point temperature of the heating material of the heating element first, and a second of the constrictions (e.g. a constriction with a greater thickness than the first constriction) may reach the Curie point temperature thereafter. Such an arrangement may be used to provide progressive heating of smokable material over time in a manner similar to that described above.

In some embodiments, the apparatus 100, 200, 300, 400, 500, 600 is sold, supplied or otherwise provided separately from the article 1, 2, 3, 4, 5, 6 with which the apparatus 100, 200, 300, 400, 500, 600 is usable. However, in some embodiments, the apparatus 100, 200, 300, 400, 500, 600 and one or more of the articles 1, 2, 3, 4, 5, 6 may be provided together as a system, such as a kit or an assembly, possibly with additional components, such as cleaning utensils.

In each of the above described embodiments, the article 1, 2, 3, 4, 5, 6 is a consumable article. Once all, or substantially all, of the volatilisable component(s) of the smokable material 25, 35, 45, 50, 55, 65 in the article 1, 2, 3, 4, 5, 6 has/have been spent, the user may remove the article 1, 2, 3, 4, 5, 6 from the apparatus 100, 200, 300, 400, 500, 600 and dispose of the article 1, 2, 3, 4, 5, 6. The user may subsequently re-use the apparatus 100, 200, 300, 400, 500, 600 with another of the articles 1, 2, 3, 4, 5, 6. However, in other respective embodiments, the article may be non-consumable, and the apparatus and the article may be disposed of together once the volatilisable component(s) of the smokable material has/have been spent.

In each of the embodiments discussed above the heating material is steel. However, in other embodiments, the heating material may comprise one or more materials selected from the group consisting of: an electrically-conductive material, a magnetic material, and a magnetic electrically-conductive material. In some embodiments, the heating material may comprise a metal or a metal alloy. In some embodiments, the heating material may comprise one or more materials selected from the group consisting of: aluminium, gold, iron, nickel, cobalt, conductive carbon, graphite, plain-carbon steel, stainless steel, ferritic stainless steel, copper, and bronze. For applications using relatively low heating temperatures, it may be advantageous to use a heating material with a lower Curie point temperature, such as an alloy comprising or consisting of iron and nickel. Example such alloys and their associated Curie point temperatures are 30%Ni-70%Fe (100 degrees Celsius), 36%Ni-64%Fe (279 degrees Celsius), and 42%Ni-58%Fe (325 degrees Celsius). Other heating material(s) may be used in other embodiments. In some embodiments, the heating element may comprise more than one material arranged as a laminate or as a composite material. The materials of the laminate or composite may be selected to optimise temperature control in use. Each of the materials of the laminate or composite may, for example, be selected from the list of materials recited earlier in this paragraph. It has been found that, when magnetic electrically-conductive material is used as the heating material, magnetic coupling between the magnetic electrically-conductive material and an electromagnet of the apparatus in use may be enhanced. In addition to potentially enabling magnetic hysteresis heating, this can result in greater or improved Joule heating of the heating material, and thus greater or improved heating of the smokable material.

The heating material may have a skin depth, which is an exterior zone within which most of an induced electrical current and/or induced reorientation of magnetic dipoles occurs. By providing that the heating material has a relatively small thickness, a greater proportion of the heating material may be heatable by a given varying magnetic field, as compared to heating material having a depth or thickness that is relatively large as compared to the other dimensions of the heating material. Thus, a more efficient use of material is achieved and, in turn, costs are reduced.

In each of the above described embodiments, the smokable material comprises tobacco. However, in respective variations to each of these embodiments, the smokable material may consist of tobacco, may consist substantially entirely of tobacco, may comprise tobacco and smokable material other than tobacco, may comprise smokable material other than tobacco, or may be free from tobacco. In some embodiments, the smokable material is a non-tobacco material which contains or comprises nicotine. In some embodiments, the smokable material may comprise a vapour or aerosol forming agent or a humectant, such as glycerol, propylene glycol, triacetin, or diethylene glycol.

In order to address various issues and advance the art, the entirety of this disclosure shows by way of illustration and example various embodiments in which the claimed invention may be practised and which provide for superior heating elements for use with apparatus for heating smokable material to volatilise at least one component of the smokable material, superior articles for use with apparatus for heating smokable material to volatilise at least one component of the smokable material, superior apparatus for heating smokable material to volatilise at least one component of the smokable material, and superior systems comprising such an article and such apparatus. The advantages and features of the disclosure are of a representative sample of embodiments only, and are not exhaustive and/or exclusive. They are presented only to assist in understanding and teach the claimed and otherwise disclosed features. It is to be understood that advantages, embodiments, examples, functions, features, structures and/or other aspects of the disclosure are not to be considered limitations on the disclosure as defined by the claims.

Various embodiments may suitably comprise, consist of, or consist in essence of, various combinations of the disclosed elements, components, features, parts, steps, means, etc. The disclosure may include other inventions not presently claimed, but which may be claimed in future.

## Claims

1. Apparatus (400, 500) for heating smokable material (45, 55) to volatilise at least one component of the smokable material, the apparatus comprising:
a heating zone (111) for receiving an article (4, 5) comprising smokable material;
a heating element (130) made of heating material that is heatable by penetration with a varying magnetic field to heat the heating zone; and
a magnetic field generator (112) for generating one or more varying magnetic fields that penetrate the heating element in use in such a way that a strength of the magnetic field, or a strength of a sum of the magnetic fields, differs at respective different locations in the heating element such that, in use, volatilisation of at least one component of the smokable material adjacent a first portion of the heating element is initiated before initiation of volatilisation of at least one component of the smokable material adjacent a second portion of the heating element;
**characterised in that** the apparatus comprises:
a detector for detecting an electrical current in the magnetic field generator; and
a controller (117) for controlling operation of the magnetic field generator on the basis of a change in the electrical current detected by the detector.

2. The apparatus of claim 1, wherein the heating element projects into the heating zone.

3. The apparatus of claim 1 or 2, wherein the heating element extends at least partially around the heating zone.

4. Apparatus (600) for heating smokable material (65) to volatilise at least one component of the smokable material, the apparatus comprising:
a heating zone (111) for receiving an article (6), the article comprising smokable material and heating material (61) that is heatable by penetration with a varying magnetic field to heat the smokable material: and
a magnetic field generator (112) for generating one or more varying magnetic fields that penetrate the heating zone in use in such a way that a strength of the magnetic field, or a strength of a sum of the magnetic fields, differs at respective different locations in the heating zone such that, in use, volatilisation of at least one component of the smokable material adjacent a first portion of the heating zone is initiated before initiation of volatilisation of at least one component of the smokable material adjacent a second portion of the heating zone;
**characterised in that** the apparatus comprises:
a detector for detecting an electrical current in the magnetic field generator; and
a controller (117) for controlling operation of the magnetic field generator on the basis of a change in the electrical current detected by the detector.

5. The apparatus of any one of claims 1 to 4, wherein the magnetic field generator comprises a first coil (114) for generating a first of the one or more varying magnetic fields, and a second coil (115) for generating a second of the one or more varying magnetic fields, and wherein the second varying magnetic field overlaps the first varying magnetic field.

6. The apparatus of claim 5, wherein the first coil is a first helical coil, and wherein the second coil is a second helical coil.

7. The apparatus of claim 6, wherein the first helical coil extends along a first axis, and wherein the second helical coil extends along a second axis that is coincident with the first axis.

8. The apparatus of any one of claims 5 to 7, wherein the second coil surrounds only a section of the first coil.

9. A system (4000, 5000, 6000) for heating smokable material (45, 55, 65) to volatilise at least one component of the smokable material, the system comprising:
the apparatus (400, 500, 600) of any one of claims 1 to 8; and
the article (4, 5, 6) for locating in the heating zone (111) of the apparatus.

## Patentansprüche

1. Vorrichtung (400, 500) zur Erwärmung von rauchbarem Material (45, 55), um zumindest eine Komponente des rauchbaren Materials zu verdampfen, wobei die Vorrichtung umfasst:
eine Erhitzungszone (111)zum Aufnehmen eines Artikels (4, 5), welcher rauchbares Material umfasst;
ein Heizelement (130), welches aus Heizmaterial hergestellt ist, welches durch Durchdringen mit einem veränderlichen Magnetfeld erhitzbar ist; und
einen Magnetfeldgenerator (112) zum Generieren eines oder mehrerer veränderlicher Magnetfelder, die das Heizelement im Gebrauch derart durchdringen, dass sich eine Stärke des Magnetfelds oder eine Stärke einer Summe der Magnetfelder an jeweiligen unterschiedlichen Stellen im Heizelement derart unterscheidet, dass im Gebrauch die Verdampfung von zumindest einer Komponente des rauchbaren Materials neben einem ersten Abschnitt des Heizelements vor der Einleitung der Verdampfung von zumindest einer Komponente des rauchbaren Materials neben einem zweiten Abschnitt des Heizelements eingeleitet wird;
**dadurch gekennzeichnet, dass** die Vorrichtung umfasst:
einen Detektor zum Detektieren eines elektrischen Stroms im Magnetfeldgenerator; und
eine Steuereinheit (117) zum Steuern des Betriebs des Magnetfeldgenerators auf der Grundlage einer vom Detektor detektierten Änderung des elektrischen Stroms.

2. Vorrichtung nach Anspruch 1, wobei das Heizelement in die Erhitzungszone hineinragt.

3. Vorrichtung nach Anspruch 1 oder 2, wobei sich das Heizelement zumindest teilweise um die Erhitzungszone herum erstreckt.

4. Vorrichtung (600) zur Erwärmung von rauchbarem Material (65), um zumindest eine Komponente des rauchbaren Materials zu verdampfen, wobei die Vorrichtung umfasst:
eine Erhitzungszone (111) zum Aufnehmen eines Artikels (6), wobei der Artikel rauchbares Material und Heizmaterial (61) umfasst, welches durch Durchdringen mit einem veränderlichen Magnetfeld erhitzbar ist, um das rauchbare Material zu erhitzen: und
einen Magnetfeldgenerator (112) zum Generieren eines oder mehrerer veränderlicher Magnetfelder, welche die Erhitzungszone bei Gebrauch derart durchdringen, dass sich eine Stärke eines Magnetfelds oder eine Stärke einer Summe der Magnetfelder an jeweiligen unterschiedlichen Stellen in der Erhitzungszone derart unterscheidet, dass bei Gebrauch die Verdampfung von zumindest einer Komponente des rauchbaren Materials neben einem ersten Abschnitt der Erhitzungszone vor der Einleitung der Verdampfung von zumindest einer Komponente des rauchbaren Materials neben einem zweiten Abschnitt der Erhitzungszone eingeleitet wird;
**dadurch gekennzeichnet, dass** die Vorrichtung umfasst:
einen Detektor zum Detektieren eines elektrischen Stroms im Magnetfeldgenerator; und
eine Steuereinheit (117) zum Steuern des Betriebs des Magnetfeldgenerators auf der Grundlage einer vom Detektor detektierten Änderung des elektrischen Stroms.

5. Vorrichtung nach einem der Ansprüche 1 bis 4, wobei der Magnetfeldgenerator eine erste Spule (114) zum Generieren eines ersten des einen oder der mehreren veränderlichen Magnetfelder und eine zweite Spule (115) zum Generieren eines zweiten des einen oder der mehreren veränderlichen Magnetfelder umfasst, und wobei das zweite veränderliche Magnetfeld das erste veränderliche Magnetfeld überlagert.

6. Vorrichtung nach Anspruch 5, wobei die erste Spule eine erste spiralförmige Spule ist, und wobei die zweite Spule eine zweite spiralförmige Spule ist.

7. Vorrichtung nach Anspruch 6, wobei sich die erste spiralförmige Spule entlang einer ersten Achse erstreckt, und wobei sich die zweite spiralförmige Spule entlang einer zweiten Achse erstreckt, die mit der ersten Achse zusammenfällt.

8. Vorrichtung nach einem der Ansprüche 5 bis 7, wobei die zweite Spule nur einen Teilabschnitt der ersten Spule umgibt.

9. System (4000, 5000, 6000) zur Erwärmung von rauchbarem Material (45, 55, 65), um zumindest eine Komponente des rauchbaren Materials zu verdampfen, wobei das System umfasst:
die Vorrichtung (400, 500, 600) nach einem der Ansprüche 1 bis 8; und
den Artikel (4, 5, 6) zum Anordnen in der Erhitzungszone (111) der Vorrichtung.

## Revendications

1. Appareil (400, 500) de chauffage de matériau à fumer (45, 55) afin de volatiliser au moins un composant du matériau à fumer, l'appareil comprenant :
une zone de chauffe (111) destinée à recevoir un article (4, 5) comprenant un matériau à fumer ;
un élément chauffant (130) constitué d'un matériau chauffant qui peut être chauffé par pénétration d'un champ magnétique variable afin de chauffer la zone de chauffage ; et
un générateur de champ magnétique (112) destiné à générer un ou plusieurs champs magnétiques variables qui pénètrent dans l'élément chauffant en cours d'utilisation de telle sorte qu'une intensité du champ magnétique, ou une intensité d'une somme des champs magnétiques, diffère à différents emplacements respectifs dans l'élément chauffant de telle sorte que, lors de l'utilisation, la volatilisation d'au moins un composant du matériau à fumer adjacent à une première partie de l'élément chauffant soit initiée avant l'initiation de la volatilisation d'au moins un composant du matériau à fumer adjacent à une deuxième partie de l'élément chauffant ;
**caractérisé en ce que** l'appareil comprend :
un détecteur pour détecter un courant électrique dans le générateur de champ magnétique ; et
un dispositif de commande (117) pour commander le fonctionnement du générateur de champ magnétique sur la base d'un changement dans le courant électrique détecté par le détecteur.

2. Appareil selon la revendication 1, dans lequel l'élément chauffant fait saillie dans la zone de chauffage.

3. Appareil selon la revendication 1 ou la revendication 2, dans lequel l'élément chauffant s'étend au moins partiellement autour de la zone de chauffage.

4. Appareil (600) de chauffage de matériau à fumer (65) afin de volatiliser au moins un composant du matériau à fumer, l'appareil comprenant :
une zone de chauffe (111) destinée à recevoir un article (6), l'article comprenant un matériau à fumer et un matériau chauffant (61) qui peut être chauffé par pénétration avec un champ magnétique variable afin de chauffer le matériau à fumer : et
un générateur de champ magnétique (112) destiné à générer un ou plusieurs champs magnétiques variables qui pénètrent dans la zone de chauffage en cours d'utilisation de telle sorte
qu'une intensité du champ magnétique, ou une intensité d'une somme des champs magnétiques, diffère à différents emplacements respectifs dans la zone de chauffage, de telle sorte que, en utilisation, la volatilisation d'au moins un composant du matériau à fumer adjacent à une première partie de la zone de chauffage soit initiée avant l'initiation de la volatilisation d'au moins un composant du matériau à fumer adjacent à une deuxième partie de la zone de chauffage ;
**caractérisé en ce que** l'appareil comprend :
un détecteur pour détecter un courant électrique dans le générateur de champ magnétique ; et
un dispositif de commande (117) pour commander le fonctionnement du générateur de champ magnétique sur la base d'un changement dans le courant électrique détecté par le détecteur.

5. Appareil selon l'une quelconque des revendications 1 à 4, dans lequel le générateur de champ magnétique comprend une première bobine (114) pour générer un premier des un ou plusieurs champs magnétiques variables, et une deuxième bobine (115) pour générer un deuxième des un ou plusieurs champs magnétiques variables, et dans lequel le deuxième champ magnétique variable chevauche le premier champ magnétique variable.

6. Appareil selon la revendication 5, dans lequel la première bobine est une première bobine hélicoïdale, et dans lequel la deuxième bobine est une deuxième bobine hélicoïdale.

7. Appareil selon la revendication 6, dans lequel la première bobine hélicoïdale s'étend le long d'un premier axe, et dans lequel la deuxième bobine hélicoïdale s'étend le long d'un deuxième axe qui coïncide avec le premier axe.

8. Appareil selon l'une quelconque des revendications 5 à 7, dans lequel la deuxième bobine n'entoure qu'une partie de la première bobine.

9. Système (4000, 5000, 6000) de chauffage de matériau à fumer (45, 55, 65) afin de volatiliser au moins un composant du matériau à fumer, le système comprenant :
l'appareil (400, 500, 600) selon l'une quelconque des revendications 1 à 8 ; et
l'article (4, 5, 6) destiné à être placé dans la zone de chauffe (111) de l'appareil.
